# EUROPEAN PATENT SPECIFICATION

(11) **EP 1 988 944 B1**
(45) Date of publication and mention of the grant of the patent: **12.05.2010**
(21) Application number: 07712806.4
(22) Date of filing: 28.02.2007
(51) Int. Cl.: A61L 27/44, A61F 2/30

(54) **ASSEMBLY COMPRISING COMPOSITE MATERIALS FOR BEARING SURFACES AND USES THEREOF IN RECONSTRUCTIVE OR ARTIFICIAL JOINTS**
ANORDNUNG AUS KOMPOSIT-MATERIALIEN FÜR TRAGEFLÄCHEN UND IHRE VERWENDUNGEN IN REKONSTRUKTIVEN ODER KÜNSTLICHEN GELENKEN
ENSEMBLE COMPRENANT DES MATÉRIAUX COMPOSITES POUR SUPPORTER DES SURFACES ET SES UTILISATIONS DANS DES ARTICULATIONS DE RECONSTRUCTION OU ARTIFICIELLES

(30) Priority: 01.03.2006 GB 0604061
(43) Date of publication of application: 12.11.2008
(73) Proprietor: Invibio Limited, Lancashire FY5 4QD (GB)
(72) Inventor: DEVINE, John, Neil, Lancashire FY6 7EE (GB)
(74) Representative: Brierley, Anthony Paul
(86) International application number: PCT/GB2007/000689
(87) International publication number: WO 2007/099307

(56) References cited:
- GB-A- 2 319 962
- US-A- 5 326 354
- US-A1- 2002 111 691
- US-A1- 2005 021 149
- WANG A ET AL: "CARBON FIBER REINFORCED POLYETHER ETHER KETONE COMPOSITE AS A BEARING SURFACE FOR TOTAL HIP REPLACEMENT" TRIBOLOGY INTERNATIONAL, BUTTERWORTH SCIENTIFIC LDT, GUILDFORD,, GB, vol. 31, no. 11, November 1998 (1998-11), pages 661-667, XP001083644 ISSN: 0301-679X cited in the application

## Description

This invention relates to polymeric materials and particularly, although not exclusively, relates to the use of such materials in assemblies comprising first and second parts which bear against one another. Preferred embodiments relate to the use of composite materials for bearing surfaces, for example for reconstructive joints (or other parts) of human bodies.

A wide range of materials has been proposed for use in reconstructive or artificial joints (or other parts) of human bodies, for example for joints or bearing surfaces in the spine; for shoulder or finger joints; and for partial or total hip or knee replacements.

Tribiology International Vol. 31, No. 11, pp 661-667, 1998 (Wang) describes the success of total hip arthroplasty in the second half of the 20^{th} century as owing greatly to the use of ultra-high molecular weight polyethylene as a bearing surface for the acetabular component. Excellent wear is acknowledged when a polyethylene bearing surface is coupled with a metal or ceramic femoral head. However, a problem is acknowledged in that the debris produced by wear of polyethylene may cause adverse biological reaction, leading to bone loss or osteolysis, and, subsequently, the need to undertake revision surgery.

Metal-on-metal articulation joints have been proposed and used with mixed results. Some metal implants may fail in a relatively short time whilst some will last much longer. Such inconsistent performance is, of course, unacceptable. It may stem from difficulties in controlling manufacturing tolerance of a metal-on-metal implant such as clearance, sphericity, surface finish or the quality of the alloy itself.

Ceramic-on-ceramic joints have been proposed but these require even higher manufacturing precision than metal-on-metal joints because of the inherent brittleness of the ceramic.

Thus metal-on-metal and ceramic-on-ceramic joints are much less forgiving in the design and manufacturing areas and more sensitive to surgical techniques compared to polyethylene/metal joints.

Another problem with known materials is the tendency for them to exhibit increased wear as the load on them increases. If, in a joint, there is anything other than a perfect fit between two bearing parts, the joint may wear more quickly than expected (due to the increased load), leading to premature failure of the joint.

It is an object of the present invention to address the aforementioned problems.

According to a first aspect of the invention, there is provided an assembly as set forth in claim 1.

Said first and second parts may bear against one another so that, in use, one or both of the parts may have a tendency to wear and/or produce wear debris by virtue of contact between the parts. Advantageously, however, the materials from which the first and second parts are made may be such that the amount of wear debris produced and the rate of wear is significantly less than for corresponding parts made from other polymeric materials such as acetal or ultra-high molecular weight polyethylene.

Preferably, a bearing surface of said first part which comprises said first composite material contacts a bearing surface of said second part which comprises said second composite material. Thus, in the assembly, a bearing surface which comprise said first composite material suitably contacts a bearing surface which comprises said second composite material.

In the assembly, said first part and said second part are preferably movable relative to one another. For example, a bearing surface of one of the parts may be arranged to slide over a bearing surface of the other part. Said first and second parts may be pivotable relative to one another.

Said first and second parts are preferably lubricated in use. For example they may be lubricated by synovial fluid when used in a human body; or lubricated by a lubrication fluid such as an oil, when used in other applications. Many different types of assemblies comprising first and second parts as described may be provided. Preferably, said assembly is for implantation in a human body, suitably to replace a structural element of the human body. Said assembly may be for use in or around the spine, for example in spinal non-fusion technologies; or for use in artificial joints, for example in fingers, hips, knees, shoulders, elbows, toes and ankles.

One of said first or second parts of the assembly may comprise a male element and the other of said first or second parts may comprise a female element wherein said male and female elements bear against one another, suitably with said bearing surfaces which comprise said first composite material and said second composite material in contact, and said male element is pivotable relative to the female element.

Said first part may be made substantially entirely from said first composite material. Alternatively, a first part may comprise a material other than said first composite material but a bearing surface of such a first part may be defined by said first composite material. Such a bearing surface may be defined by capping or coating, or otherwise providing, a layer of first composite material on a precursor of said first part for defining said first part. For example, said first part may comprise a metal or ceramic part (e.g. a femoral head) which is capped with said first composite material or the first part may comprise bone (i.e. the natural bearing material) wherein a bearing surface is capped or otherwise resurfaced with said first composite material.

Said second part may be made substantially entirely from said second composite material. Alternatively, a second part may comprise a material other than said second composite material but a bearing surface of such a second part may be defined by said second composite material. Such a bearing surface may be defined by capping or coating, or otherwise providing, a layer of second composite material on a precursor of said second part for defining said second part. For example, said second part may comprise a metal or ceramic part (e.g. a femoral head) which is capped with said second composite material or the second part may comprise bone (i.e. the natural bearing material) wherein a bearing surface is capped or otherwise resurfaced with said second composite material.

One of said first or second parts of the assembly may define a head and the other part may define a socket within which the head is pivotable.

In a preferred embodiment, said assembly may be for a hip replacement. It may comprise a femoral head and an acetabular component. Bearing surfaces which contact one another suitably are defined by said first composite material and said second composite material.

Said first polymeric material preferably includes a said repeat unit I wherein t and v independently represent 0 or 1. Preferred polymeric materials have a said repeat unit wherein either t=1 or v=0; t=0 and v=0; or t=0 and v=1. More preferred have t=1 and v=0; or t=0 and v=0. The most preferred has t=1 and v=0.

Said first polymeric material preferably includes at least 60 mole%, more preferably at least 90 mole% of repeat units of formula I. Preferably, said first polymeric material consists essentially of repeat units of formula I. Preferably, said first polymeric material includes a single type of repeat unit of formula I.

In preferred embodiments, said first polymeric material is selected from polyetheretherketone, polyetherketone and polyetherketoneketone. In a more preferred embodiment, said first polymeric material is selected from polyetherketone and polyetheretherketone. In an especially preferred embodiment, said first polymeric material is polyetheretherketone.

Thus, preferably, said first polymeric material consists essentially of a repeat unit of formula I wherein t = 1 and v = 0.

Said first polymeric material suitably has a melt viscosity (MV) of at least 0.06 kNsm⁻², preferably has a MV of at least 0.09 kNsm⁻², more preferably at least 0.12 kNsm⁻², especially at least 0.15 kNsm⁻².

MV is suitably measured using capillary rheometry operating at 400°C at a shear rate of 1000s⁻¹ using a tungsten carbide die, 0.5x3.175mm.

Said first polymeric material may have a MV of less than 1.00 kNsm⁻², preferably less than 0.5 kNsm⁻².

Said first polymeric material may have a MV in the range 0.09 to 0.5 kNsm⁻², preferably in the range 0.14 to 0.5 kNsm⁻².

Said first composite material may have an MV in the range 0.5 to 1.0 kNsm⁻², preferably in the range 0.7 to 1.0 kNsm⁻². MV may be measured by capillary rheometry.

Said first polymeric material may have a tensile strength, measured in accordance with ASTM D790 of at least 40 MPa, preferably at least 60 MPa, more preferably at least 80 MPa. The tensile strength is preferably in the range 80-110 MPa, more preferably in the range 80-100 MPa.

Said first composite material may have a tensile strength, measured in accordance with ASTM D790 of greater than 100 MPa, preferably of greater than 120 MPa.

Said first polymeric material may have a flexural strength, measured in accordance with ASTM D790 of at least 145 MPa. The flexural strength is preferably in the range 145-180 MPa, more preferably in the range 145-165 MPa.

Said first composite material may have a flexural strength, measured in accordance with ASTM D790, of at least 200 MPa.

Said first polymeric material may have a flexural modulus, measured in accordance with ASTM D790, of at least 2 GPa, preferably at least 3GPa, more preferably at least 3.5 GPa. The flexural modulus is preferably in the range 3.5-4.5 GPa, more preferably in the range 3.5-4.1 GPa.

Said first composite material may have a flexural modulus, measured in accordance with ASTM D790, of at least 7 GPa.

Advantageously, the first polymeric material and said carbon fibre may be selected to tailor the properties of the first composite material. For example, the flexural modulus may be tailored to that of cortical bone (approximately 18GPa).

Said second polymeric material preferably includes a said repeat unit I wherein t and v independently represent 0 or 1. Preferred polymeric materials have a said repeat unit wherein either t=1 or v=0; t=0 and v=0; or t=0 and v=1. More preferred have t=1 and v=0; or t=0 and v=0. The most preferred has t=1 and v=0.

Said second polymeric material preferably includes at least 60 mole%, more preferably 90 mole% of repeat units of formula I. Preferably, said second polymeric material consists essentially of repeat units of formula I. Preferably, said second polymeric material includes a single type of repeat unit of formula I.

In preferred embodiments, said second polymeric material is selected from polyetheretherketone, polyetherketone and polyetherketoneketone. In a more preferred embodiment, said second polymeric material is selected from polyetherketone and polyetheretherketone. In an especially preferred embodiment, said second polymeric material is polyetheretherketone.

Thus, preferably, said second polymeric material consists essentially of a repeat unit of formula I wherein t = 1 and v = 0.

Said second polymeric material suitably has a melt viscosity (MV) of at least 0.06 kNsm⁻², preferably has a MV of at least 0.09 kNsm⁻², more preferably at least 0.12 kNsm⁻², especially at least 0.15 kNsm⁻².

MV is suitably measured using capillary rheometry operating at 400°C at a shear rate of 1000s⁻¹ using a tungsten carbide die, 0.5x3.175mm.

Said second polymeric material may have a MV of less than 1.00 kNsm⁻², preferably less than 0.5 kNsm⁻².

Said second polymeric material may have a MV in the range 0.09 to 0.5 kNsm⁻², preferably in the range 0.14 to 0.5 kNsm⁻².

Said second composite material may have an MV in the range 0.5 to 1.0 kNsm⁻², preferably in the range 0.7 to 1.0 kNsm⁻².

Said second polymeric material may have a tensile strength, measured in accordance with ASTM D790 of at least 40 MPa, preferably at least 60 MPa, more preferably at least 80 MPa. The tensile strength is preferably in the range 80-110 MPa, more preferably in the range 80-100 MPa.

Said second composite material may have a tensile strength, measured in accordance with ASTM D790 of greater than 100 MPa, preferably of greater than 120 MPa.

Said second polymeric material may have a flexural strength, measured in accordance with ASTM D790 of at least 145 MPa. The flexural strength is preferably in the range 145-180 MPa, more preferably in the range 145-165 MPa.

Said second composite material may have a flexural strength, measured in accordance with ASTM D790, of at least 200 MPa.

Said second polymeric material may have a flexural modulus, measured in accordance with ASTM D790, of at least 2 GPa, preferably at least 3GPa, more preferably at least 3.5 GPa. The flexural modulus is preferably in the range 3.5-4.5 GPa, more preferably in the range 3.5-4.1 GPa.

Said second composite material may have a flexural modulus, measured in accordance with ASTM D790, of at least 7 GPa.

Advantageously, the second polymeric material and said carbon fibre may be selected to tailor the properties of the second composite material. For example, the flexural modulus may be tailored to that of cortical bone (approximately 18GPa).

Preferably, said first polymeric material and said second polymeric material are the same.

Said carbon fibre may be of any suitable type. Said carbon fibre may be PAN-based or pitch based.

Suitable PAN-based fibres may have a fibre density in the range 1.7 to 1.85 g.cm⁻³, a tensile strength of greater than 2900 MPa, a tensile modulus in the range 230-250 GPa a bulk density of greater than 350 g/l.

Suitable pitch-based carbon fibres may have a fibre density in the range 1.2-2 g.cm⁻³, a tensile strength in the range 400-600 MPa and a Young's Modulus of 30-50GPa.

Said carbon fibre may comprise milled forms, for example having average lengths in the range 200-1600µm. Alternatively, the carbon fibres may be in chopped lengths for example having average lengths in the range 3 to 30mm. In a further alternative, endless carbon fibres may be present in the first and/or second composite materials. Such endless materials may comprise 6000 or 12000 filament tows.

The carbon fibres may incorporate additives or a finish as is conventional for such materials to improve compatibility of the fibres with the first and second polymeric materials.

Preferably, said first part comprises a first composite material comprising said first polymeric material and PAN-based carbon fibres. Preferably PAN-based fibres make up at least 50wt%, at least 75wt%, at least 90wt%, at least 95wt%, especially about 100wt% of the carbon fibre of the first composite material. Preferably, said second part comprises said second composite material comprising said second polymeric material and PAN-based carbon fibres. Preferably PAN-based fibres make up at least 50wt%, at least 75wt%, at least 90wt%, at least 95wt%, especially about 100wt% of the carbon fibre of the second composite material.

Said first composite material suitably includes at least 30 wt%, preferably at least 45 wt%, more preferably at least 60 wt%, especially at least 65wt% of said first polymeric material. Said composite material may include up to 70wt%, up to 55wt%, up to 40wt%, up to 35wt% of carbon fibres. Said first composite material may include 30 to 70wt% of said first polymeric material and 30 to 70wt% of carbon fibre. In a preferred embodiment said first composite material comprises 60 to 80 wt% of polymeric material of formula I, preferably of formula I wherein t = 1 and v = 0, and 20 to 40 wt% of carbon fibre.

Said first composite material may include one or more further components. It may include up to 15wt%, preferably up to 10wt% of other components. An example of another component is an X-ray contrast material for example barium sulphate.

Said first composite material preferably includes only a single type of first polymeric material of formula I. It may also include only a single type of carbon fibre - e.g. only PAN-based; or only pitch-based, but not a mixture of two types.

Said carbon fibre of the second composite material may independently have any features of the carbon fibre of the first composite material.

Said second composite material suitably includes at least 30 wt%, preferably at least 45 wt%, more preferably at least 60 wt%, especially at least 65wt%, of said second polymeric material. Said composite material may include up to 70wt%, up to 55wt%, up to 40wt%, up to 35wt% of carbon fibre. Said second composite material may include 30 to 70wt% of said second polymeric material and 30 to 70wt% of carbon fibre. In a preferred embodiment said second composite material comprises 60 to 80 wt% of polymeric material of formula I, preferably of formula I wherein t = 1 and v = 0, and 20 to 40 wt% of carbon fibre.

Said second composite material may include one or more further components. It may include up to 15wt%, preferably up to 10wt% of other components. An example of another component is an X-ray contrast material for example barium sulphate.

Said second composite material preferably includes only a single type of second polymeric material of formula I. It may also include only a single type of carbon fibre - e.g. only PAN-based; or only pitch-based, but not a mixture of two types.

Said first composite material and said second composite material preferably comprise the same polymeric material of formula I and preferably the same carbon fibre. Preferably, said first composite material and said second composite material have substantially the same composition.

The assembly of the first aspect may include one or more additional parts which may bear against said first and/or said second parts. Said one or more additional parts may be made from a said first composite material as described.

According to a second aspect of the invention, there is provided a kit for providing an assembly of said first aspect, the kit comprising:
(a) a first part as described according to said first aspect;
(b) a second part as described according to said first aspect;
wherein said first part and said second part are cooperable to define an assembly wherein said first and second parts bear against one another.

Said first part and said second part may have any feature of the first part and the second part of the first aspect *mutatis mutandis*.

According to a third aspect of the invention, there is provides a package, which is preferably substantially sterile, which comprises an assembly or kit according to the first or second aspects respectively.

According to a fourth aspect, there is provided a method of manufacturing a first part and a second part as described according to the first and second aspects, the method comprising forming respective bearing surfaces of said first and second parts from a first composite material and a second composite material respectively.

The method may comprise making one or both of said parts substantially entirely from said first or second composite materials; or the method may comprise forming one or both bearing surfaces (but not the entirety) of said first and second parts out of said first and/or second composite materials.

According to a fifth aspect of the invention, there is provided a method of making an assembly according to the first aspect, the method comprising:
(a) selecting a first part as described according to the first aspect;
(b) selecting a second part as described according to the first aspect; and
(c) contacting the first and second parts so that the parts bear against one another and define said assembly.

According to a sixth aspect of the invention, there is provided the use of a first part as described according to the first aspect and a second part as described according to the first aspect in the manufacture of an assembly which comprises said first and second parts bearing against one another for implantation into the human body, for example to replace a structural element of the body.

Any feature of any aspect of the invention or embodiment described herein may be combined with any other feature of any aspect of an invention or embodiment described herein *mutatis mutandis*.

Specific embodiments of the invention will now be described by way of example.

The following materials are referred to hereinafter:
PEEK-OPTIMA LT1 - Long term implantable grade polyetheretherketone with a melt viscosity of approximately 0.45kNsm⁻², obtainable from Invibio Limited, UK.
CFR-PEEK-LT1 - Implant grade polyetheretherketone containing 30% by weight PAN based carbon fibres, obtained from Invibio Limited, UK.
Acetal refers to poly(oxymethylene).
   UHMWPE - refers to Ultra High Molecular Weight Polyethylene obtained from DuPuy Orthopaedics.
   Pin-on-plate testing was used to assess materials. The pins and plates were made according to the general procedures described in Example 1 and 2 and tested using the general procedure described in Example 3.

### Example 1 - General procedure for making pins

All pins were machined from injection moulded plaques. All plaques were produced using standard conditions, for example those described in general literature available from Invibio Limited. The machined pins were polished to give a surface roughness (Sa) of approximately 1 micron. All pins were cleaned in aqueous ethanol and demineralised water and annealed using a general annealing protocol for example as described in general literature available from Invibio Ltd. All pins were machined such that any fibre alignment caused by the direction of polymer flow would be parallel with the reciprocating motion. Unless specified, all pins were gamma sterilised with an irradiation dose of 50kGy.

### Example 2 - General procedure for making plates

All plates were machined from injection moulded plaques. All plaques were produced using standard conditions. The machined plates were machined to maintain the injection moulded surface finish (Sa of approximately 0.1 micron). All plates were cleaned in aqueous ethanol and demineralised water and annealed using a general annealing protocol. All plates were machined such that any fibre alignment caused by the direction of polymer flow would be parallel with the reciprocating motion.

### Example 3 - General procedure for testing materials

A pin-on-plate machine was used. The machine was a four station pin-on-plate machine which applied both reciprocation and rotational motion. The reciprocation was applied by a sledge moving along two fixed parallel hardened steel bars and a heated bed, lubricant tray and plate holder were positioned on top of this sledge. The rotational motion was applied to each pin using a small motor. The cycle frequencies of both the reciprocation and the rotation was set at approximately 1 Hz. The plate holder consisted of four wells into which the plate specimens were clamped. A lubricant was contained within the lubricant tray and heated to a temperature of 37°C by resistors within the bed. This was controlled by a thermocouple. A load (either of 20 N or 40 N) as applied to each station via a lever arm mechanism. A lubricant level sensor made from platinum wire was attached to the lubricant tray to allow the lubricant to be maintained at an almost constant level. This was topped up from a reservoir of distilled water. An electronic counter was connected to the reciprocating sledge. Stroke length was set to 25 mm. A cover was placed over the entire rig to prevent dust contamination from the atmosphere.

The lubricant used was 24.5% bovine serum (protein content: 15 gl⁻¹) with 0.2% sodium azide added to retard the growth of bacteria and 20 mM EDTA to prevent calcium deposition. The wear was assessed gravimetrically. At least twice a week (approx. 0.25 million cycles) the machine was stopped to allow for cleaning and weighing of the samples. Any excess lubricant was cleaned from the lubricant baths and the pins and plates removed. The samples were then cleaned and dried using a predetermined and consistent protocol. The pins and plates were then weighed three times on a balance (accurate to 0.1 mg) and an average weight recorded. Control specimens were used to take account of the lubricant absorption of both the pins and plates during the test duration. The machine was then reassembled and the lubricant refreshed. The wear tests were performed up to two million cycles.

Vacuum oven drying tests were also performed both before and after the wear tests in an attempt to get the 'true' weight loss of these materials and compare this to the standard weight loss measurements.

The wear volumes were plotted against sliding distance and the gradient of the line through the data (determined by linear regression analysis) provided the wear rate. The wear rate was then divided by the load and sliding distance to determine the wear factor, k (mm³N⁻¹m⁻¹).

### Examples 4 to 12

Using the procedures described in Examples 1 and 2 pins and plates were made and combinations tested under specified loads, using the general procedure described in Example 3. A summary of materials used, the load applied and calculated wear factors is provided in Table 1. Table 2 describes volumetric wear for selected example.

**Table 1**

| **Example No.** | **Pin material** | **Plate material** | **Load (N)** | **Wear factors (mm³ N⁻¹ x 10⁻⁶** | | |
|---|---|---|---|---|---|---|
| 4 | Acetal | UHMWPE | 40N | 1.373 | 2.746 | 4.119 |
| 5 | UHMWPE | Acetal | 40N | 2.393 | 1.701 | 4.094 |
| 6 | UHMWPE | PEEK-OPTIMA LT1 (Non-Sterilised) | 40N | 5.431 | 0.529 | 5.960 |
| 7 | PEEK-OPTIMA LT1 Non-Sterilised. | UHMWPE | 40N | 0.162 | 4.163 | 4.325 |
| 8 | PEEK-OPTIMA LT1 non-sterilised | PEEK-OPTIMA LT1 non-sterilised | 40 | 2.34 | 2.33 | 4.67 |
| 9 | PEEK-OPTIMA LT1 | PEEK-OPTIMA LT1 | 40 | 1.92 | 2.58 | 4.50 |
| 10 | PEEK-OPTIMA LT1 | PEEK-OPTIMA LT1 | 20 | 2.30 | 3.56 | 5.86 |
| 11 | PEEK-OPTIMA LT1 with 30% PAN Carbon Fibres | PEEK-OPTIMA LT1 with 30% PAN Carbon Fibres | 40 | 0.07 | 0.27 | 0.34 |
| 12 | PEEK-OPTIMA LT1 with 30% PAN Carbon Fibres | PEEK-OPTIMA LT1 with 30% PAN Carbon Fibres | 20 | 0.36 | 0.53 | 0.89 |
| 13 | UHMWPE Gamma Sterilised | Stainless Steel | 40N | 1.1 | 0 | 1.1 |
| 14 | High Carbon CoCrMo | High Carbon CoCrMo | 40N | 0.78 | 0.06 | 0.84 |

**Table 2**

| **Example** No. | **Pin material** | **Plate material** | **Load (N)** | **Volumetric Wear (mm³/million cycles)** | | |
|---|---|---|---|---|---|---|
| | | | | Pin | Plate | Total |
| 10 | PEEK-OPTIMA LT1 | PEEK-OPTIMA LT1 | 20 | 2.3 | 3.68 | 5.98 |
| 9 | PEEK-OPTIMA LT1 | PEEK-OPTIMA LT1 | 40 | 3.59 | 4.97 | 8.56 |
| 12 | PEEK-OPTIMA LT1 with 30% PAN Carbon Fibres | PEEK-OPTIMA LT1 with 30% PAN Carbon Fibres | 20 | 0.35 | 0.49 | 0.84 |
| 11 | PEEK-OPTIMA LT1 with 30% PAN Carbon Fibres | PEEK-OPTIMA LT1 with 30% PAN Carbon Fibres | 40 | 0.15 | 0.53 | 0.68 |

### Example 13

By processes analogous to the processes described above, the wear performance of a composite comprising PEEK-OPTIMA LT1 and PAN carbon fibre pins and plates bearing against one other was compared to the wear performance of a composite comprising PEEK-OPTIMA LT1 and pitch-based carbon fibre pins and plates bearing against each other.

After 5 million cycles the results are as follows:

| Wear couple | | Total wear factor mm³N⁻¹mm⁻¹x10⁻⁶ |
|---|---|---|
| CFR-PEEK (PAN) | CFR-PEEK (PAN) | 0.25 |
| CFR-PEEK (Pitch) | CRF-PEEK (Pitch) | 0.92 |

### Results and discussion

Referring to Table 1, it can be seen that the total wear of the non-sterilised PEEK coupling is similar to the total wear of the sterilised PEEK coupling.

The carbon fibre-PEEK samples articulating against the same material (Examples 11 and 12) gave lower wear than the all-PEEK components (Examples 8 to 10) and indeed the lowest wear for any of the all polymeric wear couples tested. The total wear factors for the test using a 40 N load were thirteen times lower for the carbon fibre-PEEK material (Examples 11 and 12) than the PEEK samples (Examples 7 to 10) and for the test using a 20 N load, they were six times lower.

The wear factors for PEEK-OPTIMA LT1 containing 30% PAN carbon fibres articulating against the same material (example 11) has a lower wear factor than that of traditionally used successful bearing couples used in medical implants. When compared with UHMWPE articulating against metal (example 13) a greater than 60% reduction in wear factor was observed for PEEK-OPTIMA LT1 containing 30% PAN carbon fibres articulating against the same material. When compared with a metal on metal wear couple (example 14) a greater than 40% reduction in wear factor was observed for PEEK-OPTIMA LT1 containing 30% PAN carbon fibres articulating against the same material.

Referring to Table 2, the volumetric wear of PEEK-OPTIMA LT1 articulating against the same material, unsurprisingly showed that under a 40 N load higher actual wear rates were observed than when under a 20 N load. This is expected as the wear rate should increase with an increase in the applied load see T A Stolarski, Wear 1992, 158, 71-78. "Tribology of polyetheretherketone" ; SM Hosseini and TA Stolarski, Journal of Applied Polymer Science, 1992, 45, 2021-2030, "Morphology of Polymer Wear Debris Resulting from Different Contact Conditions"; MQ Zhang, ZP Lu and K Friedrich, Tribology International 1997, 30, 103-111; ZP Lu and K Friedrich, Wear 1995, 181-183, 624-631, "On sliding friction and wear of PEEK and its composites"; TJ Joyce, HE Ash and A Unsworth, Proc. Instn. Mech Engineers 1996, 210, 11, "The wear of cross-linked polyethylene against itself"; TJ Joyce and A Unsworth, Proc. Instn. Mech Engineers 1996, 210, 297, "A comparison of the wear of cross-linked polyethylene against itself with the wear of ultrahigh molecular weight polyethylene against itself". For the PEEK samples the volumetric wear rate with the 40 N load was 8.56 mm³/million cycles and for the 20 N load this was 5.98 mm³/million cycles.

However, surprisingly, the carbon fibre-PEEK components demonstrated a lower volumetric wear rate for the 40 N load (0.68 mm³/million cycles), than for the same wear couple tested under 20 N loads (0.84 mm³/million cycles).

Referring to Example 13, it appears that a wear couple comprising PEEK and PAN-based carbon fibres exhibits lower wear compared to a couple comprising Pitch-based fibres.

It should now be appreciated that, in particular, the composite materials described may advantageously be used in bearing applications - they have low wear rates and the material may advantageously be used for bearing surfaces for reconstructive joints or other parts. It should also be noted that these materials demonstrate an improvement in wear performance at increased loads and therefore there may be benefits to using these materials in high load applications such as total knee joints.

In comparison with metal or ceramic components these materials can be manufactured by a lower cost and more efficient manufacturing route such as injection moulding. There may be additional benefits in using these lower modulus materials compared with metals or ceramics, which can cause stress shielding and subsequent bone resorption.

Other advantages of the materials described are that they are less brittle than ceramics; and use of the materials avoids the production of metallic wear debris and the associated health risk of metal ions being released into the body (see for example R Michel, J Hofman, F Loer and J Zilkens "Trace element burdening of human tissues due to the corrosion of hip joint prostheses made of cobalt chrome molybdenum" and Arch. Orthop. and Traumat. Surg. 1984, 103, 85-95; and T Visuri, E Pukkala, P Paavolainen, P Pulkkinen, EB Riska, Clin Orthop 1996; 329:S280-289, wherein it is described how in patients who had a metal on metal total hip replacement the total risk of cancer was found to be 1.23 times higher than that experienced by patients with PE on metal total hip replacements).

Further advantages of the composite materials described include lower weight than metals or ceramics; and improved mechanical properties compared with UHMWPE thereby allowing thinner parts, a greater degree of motion and design flexibility.

## Claims

1. An assembly for implantation in a human body comprising:
(a) a first part which comprises a first composite material which includes a first polymeric material and carbon fibre, wherein said first polymeric material includes a repeat unit of formula 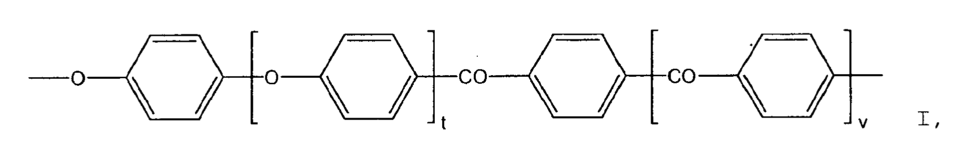 and;
(b) a second part which comprises a second composite material which includes a second polymeric material and carbon fibre, wherein said second polymeric material includes a repeat unit of formula 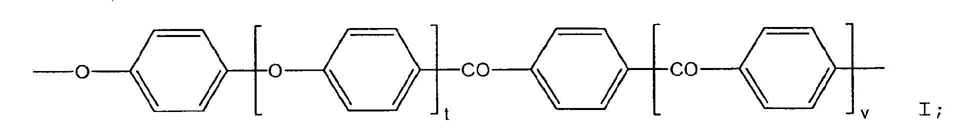 wherein said first and second parts bear against one another with a bearing surface which comprises said first composite material contacting a bearing surface which comprises said second composite material.

2. An assembly according to claim 1, wherein a bearing surface of the parts is arranged to slide over a bearing surface of the other part; or said first and second parts are pivotable relative to one another.

3. An assembly according to claim 1 or claim 2, said assembly being for use in or around the spine; or for use in an artificial joint.

4. An assembly according to any preceding claim, wherein one of said first or second parts comprises a male element and the other of said first or second parts comprises a female element wherein said male and female elements bear against one another.

5. An assembly according to any preceding claim, wherein a bearing surface of said first part which comprises said first composite material contacts a bearing surface of said second part which comprises said second composite material wherein:
said first part is made substantially entirely from said first composite material; or said first part comprises a material other than said first composite material but a bearing surface of said first part is defined by said composite material; and
said second part is made substantially entirely from said second composite material; or said second part comprises a material other than said second composite material but a bearing surface of such a second part is defined by said second composite material.

6. An assembly according to any preceding claim, wherein one of said first or second parts of the assembly defines a head and the other part defines a socket within which the head is pivotable.

7. An assembly according to any preceding claim, wherein said assembly is for a hip replacement.

8. An assembly according to any preceding claim, wherein said first polymeric material consists essentially of a repeat unit of formula I wherein t=1 and v=0 and said second polymeric material consists essentially of a repeat unit of formula I wherein t=1 and v=0.

9. An assembly according to any preceding claim, wherein said first polymeric material and said second polymeric material are the same.

10. An assembly according to any preceding claim, wherein said first composite material comprises 60 to 80wt% of polymeric material of formula I and 20 to 40wt% of carbon fibre; and said second composite material comprises 60 to 80wt% of polymeric material of formula I and 20 to 40wt% of carbon fibre.

11. An assembly according to any preceding claim, wherein said first part comprises a first composite material comprising said first polymeric material and PAN-based carbon fibres and said second part comprises said second composite material comprising said second polymeric material and PAN-based carbon fibres.

12. A kit for providing an assembly of any preceding claim, the kit comprising:
(c) a first part as described according to any of claims 1 to 11; and
(d) a second part as described according to any of claims 1 to 11;
wherein said first part and said second part are cooperable to define an assembly wherein said first and second parts bear against one another.

13. A method of manufacturing a first part and a second part as described according to any of claims 1 to 11, the method comprising forming respective bearing surfaces of said first and second parts from a first composite material and a second composite material respectively.

14. A method of making an assembly according to any of claims 1 to 11, the method comprising:
(a) selecting a first part as described in any of claims 1 to 11;
(b) selecting a second part as described in any of claims 1 to 11; and
(c) contacting the first and second parts so that the parts bear against one another and define said assembly.

15. The use of a first part according to any of claims 1 to 11 and a second part according to any of claims 1 to 11 in the manufacture of an assembly which comprises said first and second part bearing against one another for implantation into the human body.

## Patentansprüche

1. Aufbau zur Implantation in einen menschlichen Körper, der umfasst:
(a) ein erstes Teil, welches ein erstes Kompositmaterial umfasst, welches ein erstes polymeres Material und Kohlefaser beinhaltet, wobei das erste polymere Material eine Wiederholungseinheit der Formel 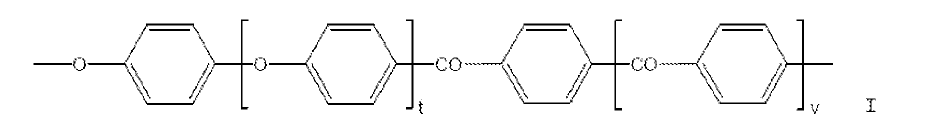 beinhaltet, und;
(b) ein zweites Teil, welches ein zweites Kompositmaterial umfasst, welches ein zweites polymeres Material und Kohlefaser beinhaltet, wobei das zweite polymere Material eine Wiederholungseinheit der Formel 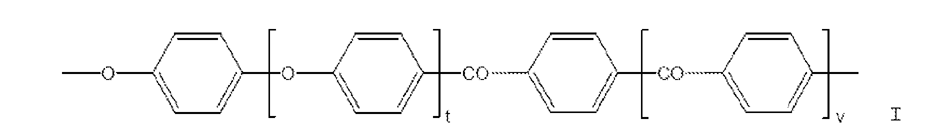 beinhaltet;
wobei das erste und das zweite Teil aneinander anliegen, mit einer Auflagefläche, welche das erste Kompositmaterial umfasst, die eine Auflagefläche, welche das zweite Kompositmaterial umfasst, kontaktiert.

2. Aufbau nach Anspruch 1, wobei eine Auflagefläche eines der Teile so eingerichtet ist, dass sie über eine Auflagefläche des anderen Teils gleitet; oder das erste und das zweite Teil relativ zueinander drehbar sind.

3. Aufbau nach Anspruch 1 oder Anspruch 2, wobei der Aufbau für die Verwendung in oder um die Wirbelsäule; oder für die Verwendung in einem künstlichen Gelenk ist.

4. Aufbau nach einem der vorhergehenden Ansprüche, wobei eines aus dem ersten und dem zweiten Teil ein männliches Element umfasst und das andere aus dem ersten und dem zweiten Teil ein weibliches Element umfasst, wobei das männliche und das weibliche Element aneinander anliegen.

5. Aufbau nach einem der vorhergehenden Ansprüche, wobei eine Auflagefläche des ersten Teils, welche das erste Kompositmaterial umfasst, eine Auflagefläche des zweiten Teils, welche das zweite Kompositmaterial umfasst, kontaktiert, wobei:
das erste Teil im Wesentlichen gänzlich aus dem ersten Kompositmaterial hergestellt ist; oder das erste Teil ein anderes Material als das erste Kompositmaterial umfasst, aber eine Auflagefläche des ersten Teils durch das Kompositmaterial definiert ist; und
das zweite Teil im Wesentlichen gänzlich aus dem zweiten Kompositmaterial hergestellt ist; oder das zweite Teil ein anderes Material als das zweite Kompositmaterial umfasst, aber eine Auflagefläche des zweiten Teils durch das zweite Kompositmaterial definiert ist.

6. Aufbau nach einem der vorhergehenden Ansprüche, wobei eines aus dem ersten und dem zweiten Teil des Aufbaus einen Kopf definiert und das andere Teil eine Pfanne definiert, in welcher der Kopf drehbar ist.

7. Aufbau nach einem der vorhergehenden Ansprüche, wobei der Aufbau für einen Hüftersatz ist.

8. Aufbau nach einem der vorhergehenden Ansprüche, wobei das erste polymere Material im Wesentlichen aus einer Wiederholungseinheit der Formel I besteht, wobei t=1 und v=0 ist, und das zweite polymere Material im Wesentlichen aus einer Wiederholungseinheit der Formel I besteht, wobei t=1 und v=0 ist.

9. Aufbau nach einem der vorhergehenden Ansprüche, wobei das erste polymere Material und das zweite polymere Material das gleiche sind.

10. Aufbau nach einem der vorhergehenden Ansprüche, wobei das erste Kompositmaterial 60 bis 80 Gew.-% polymeres Material der Formel I und 20 bis 40 Gew.-% Kohlefaser umfasst; und das zweite Kompositmaterial 60 bis 80 Gew.-% polymeres Material der Formel I und 20 bis 40 Gew.-% Kohlefaser umfasst.

11. Aufbau nach einem der vorhergehenden Ansprüche, wobei das erste Teil ein erstes Kompositmaterial umfasst, das das erste polymere Material und PAN-basierte Kohlefasern umfasst, und das zweite Teil das zweite Kompositmaterial umfasst, das das zweite polymere Material und PAN-basierte Kohlefasern umfasst.

12. Bausatz zum Bereitstellen eines Aufbaus nach einem der vorhergehenden Ansprüche, wobei der Bausatz umfasst:
(a) ein erstes Teil, wie nach einem der Ansprüche 1 bis 11 beschrieben; und
(b) ein zweites Teil, wie nach einem der Ansprüche 1 bis 11 beschrieben;
wobei das erste Teil und das zweite Teil zusammenwirken können, um einen Aufbau zu definieren, wobei das erste und das zweite Teil aneinander anliegen.

13. Verfahren zum Anfertigen eines ersten Teils und eines zweiten Teils, wie nach einem der Ansprüche 1 bis 11 beschrieben, wobei das Verfahren das Bilden entsprechender Auflageflächen des ersten und des zweiten Teils aus einem ersten Kompositmaterial beziehungsweise einem zweiten Kompositmaterial umfasst.

14. Verfahren zum Herstellen eines Aufbaus nach einem der Ansprüche 1 bis 11, wobei das Verfahren umfasst:
(a) Auswählen eines ersten Teils, wie in einem der Ansprüche 1 bis 11 beschrieben;
(b) Auswählen eines zweiten Teils, wie in einem der Ansprüche 1 bis 11 beschrieben; und
(c) Kontaktieren des ersten und des zweiten Teils, so dass die Teile aneinander anliegen und den Aufbau definieren.

15. Verwendung eines ersten Teils nach einem der Ansprüche 1 bis 11 und eines zweiten Teils nach einem der Ansprüche 1 bis 11 bei der Anfertigung eines Aufbaus, welcher das erste und das zweite Teil aneinander anliegend umfasst, zur Implantation in den menschlichen Körper.

## Revendications

1. Ensemble pour implantation dans le corps humain comprenant :
(a) une première partie qui comprend un premier matériau composite qui comporte un premier matériau polymère et une fibre de carbone, où ledit premier matériau polymère comporte un motif de répétition de formule 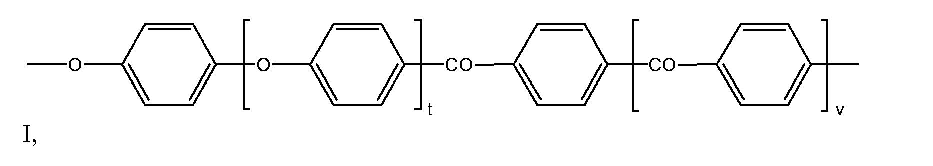 et ;
(b) une seconde partie qui comprend un second matériau composite qui comporte un second matériau polymère et une fibre de carbone, où ledit second matériau polymère comporte un motif de répétition de formule 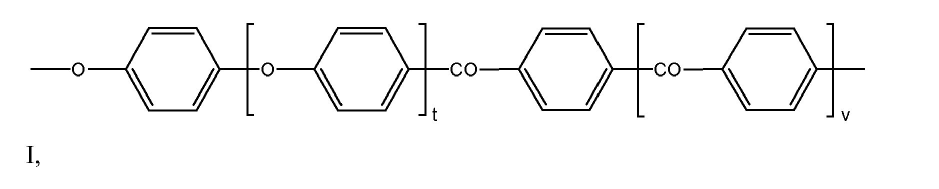
où lesdites première et seconde parties reposent l'une sur l'autre avec une surface portante qui comprend ledit premier matériau composite en contact avec une surface portante qui comprend ledit second matériau composite.

2. Ensemble selon la revendication 1, dans lequel une surface portante de l'une des parties est agencée de sorte à glisser sur une surface portante de l'autre partie ; ou bien lesdites première et seconde parties peuvent pivoter l'une par rapport à l'autre.

3. Ensemble selon la revendication 1 ou la revendication 2, ledit ensemble étant destiné à une utilisation dans ou autour de la colonne vertébrale ; ou bien à une utilisation dans une articulation artificielle.

4. Ensemble selon l'une quelconque des revendications précédentes, dans lequel l'une desdites première ou seconde parties comprend un élément mâle et l'autre desdites première ou seconde partie comprend un élément femelle où lesdits éléments mâle et femelle reposent l'une sur l'autre.

5. Ensemble selon l'une quelconque des revendications précédentes, dans lequel une surface portante de ladite première partie qui comprend ledit premier matériau composite est en contact avec une surface portante de ladite seconde partie qui comprend ledit second matériau composite où :
ladite première partie est constituée quasiment entièrement dudit premier matériau composite ; ou ladite première partie comprend un matériau autre que ledit premier matériau composite mais une surface portante de ladite première partie est définie par ledit matériau composite ; et
ladite seconde partie est constituée quasiment entièrement dudit second matériau composite ; ou ladite seconde partie comprend un matériau autre que ledit second matériau composite mais une surface portante de cette seconde partie est définie par ledit second matériau composite.

6. Ensemble selon l'une quelconque des revendications précédentes, dans lequel l'une desdites première ou seconde parties de l'ensemble définit une tête et l'autre partie définit une cavité dans laquelle la tête peut pivoter.

7. Ensemble selon l'une quelconque des revendications précédentes, dans lequel ledit ensemble est destiné à un remplacement de hanche.

8. Ensemble selon l'une quelconque des revendications précédentes, dans lequel ledit premier matériau polymère est essentiellement constitué d'un motif de répétition de formule I où t = 1 et v = 0 et ledit second matériau polymère est essentiellement constitué d'un motif répété de formule I où t = 1 et v = 0.

9. Ensemble selon l'une quelconque des revendications précédentes, dans lequel ledit premier matériau polymère et ledit second matériau polymère sont identiques.

10. Ensemble selon l'une quelconque des revendications précédentes, dans lequel ledit premier matériau composite comprend 60 à 80 % en poids de matériau polymère de formule I et 20 à 40 % en poids de fibre de carbone ; et ledit second matériau composite comprend 60 à 80 % en poids de matériau polymère de formule I et 20 à 40 % en poids de fibre de carbone.

11. Ensemble selon l'une quelconque des revendications précédentes, dans lequel ladite première partie comprend un premier matériau composite comprenant ledit premier matériau polymère et des fibres de carbone à base de PAN et ladite seconde partie comprend ledit second matériau composite comprenant ledit second matériau polymère et des fibres de carbone à base de PAN.

12. Matériel pour fournir un ensemble selon l'une quelconque des revendications précédentes, le matériel comprenant :
(a) une première partie telle que décrite selon l'une quelconque des revendications 1 à 11 ; et
(b) une seconde partie telle que décrite selon l'une quelconque des revendications 1 à 11 ;
où ladite première partie et la seconde partie peuvent coopérer pour définir un ensemble où lesdites première et seconde parties reposent l'une sur l'autre.

13. Procédé de fabrication d'une première partie et d'une seconde partie comme décrit selon l'une quelconque des revendications 1 à 11, le procédé comprenant la formation de surfaces portantes respectives desdites première et seconde parties à partir d'un premier matériau composite et d'un second matériau composite respectivement.

14. Procédé de création d'un ensemble selon l'une quelconque des revendications 1 à 1 l, le procédé comprenant :
(a) la sélection d'une première partie telle que décrite selon l'une quelconque des revendications 1 à 11 ;
(b) la sélection d'une seconde partie telle que décrite selon l'une quelconque des revendications 1 à 11 ; et
(c) la mise en contact des première et seconde parties de sorte que les parties reposent l'une sur l'autre et définissent ledit ensemble.

15. Utilisation d'une première partie selon l'une quelconque des revendications 1 à 11 et d'une seconde partie selon l'une quelconque des revendications 1 à 11 dans la fabrication d'un ensemble qui comprend lesdites première et seconde parties reposant l'une sur l'autre pour implantation dans le corps humain.
